# EUROPÄISCHE PATENTANMELDUNG

(11) **EP 4 270 415 A1**
(43) Veröffentlichungstag der Anmeldung: **01.11.2023**
(21) Anmeldenummer: 22170257.4
(22) Anmeldetag: 27.04.2022
(51) Int. Cl.: G16H 40/63, G16H 40/67, A61F 15/00, A61F 17/00

(54) **ERSTE-HILFE-STATION**

(71) Anmelder: Rhenus SN digital GmbH & Co. KG, 15366 Hoppegarten (DE)
(72) Erfinder: von Frieling, Jonas, 15566 Schöneiche bei Berlin (DE)
(74) Vertreter: Beyer, Andreas

(57) **Zusammenfassung**

Die Erfindung betrifft eine Erste-Hilfe-Station (2), die zum Erfassen und Melden einer Entnahme zumindest eines Ersthilfsmittels (8; 10; 12) ausgerüstet ist. Die Erste-Hilfe-Station (2) umfasst eine Halteeinrichtung (6) zum Halten zumindest eines Ersthilfsmittels (8; 10; 12) in der bzw. an der Erste-Hilfe-Station (2), eine Überwachungseinrichtung (16) zum Erfassen einer Entnahme des zumindest einen Ersthilfsmittels (8; 10; 12) aus der bzw. von der Erste-Hilfe-Station (2), und eine Kommunikationseinrichtung (46) zum Melden einer erfassten Entnahme über eine digitale Kommunikationsverbindung (48) an eine Überwachungsstelle. Die Erfindung betrifft ferner ein Rechensystem (4) zum Verarbeiten von Meldungen der Erste-Hilfe-Station (2).

## Beschreibung

Die vorliegende Erfindung betrifft eine Erste-Hilfe-Station sowie ein Rechensystem. Sie betrifft insbesondere eine Erste-Hilfe-Station, die zum Erfassen und Melden einer Entnahme eines Ersthilfsmittels an eine Überwachungsstelle ausgerüstet ist, sowie ein Rechensystem zum Empfangen einer solchen Meldung.

Erste-Hilfe-Stationen dienen üblicherweise der Unterstützung von Hilfe leistenden Personen. Hierzu sind derartige Stationen meist mit einem oder mehreren Ersthilfsmitteln ausgestattet, das bzw. die von einer Hilfe leistenden Person aus der Erste-Hilfe-Station entnommen werden kann. Ein derartiges Ersthilfsmittel dient in der Regel der Durchführung einer Sofortmaßnahme, die insbesondere eine Lebensrettung und/oder Gesundheitserhaltung bezweckt. Das Ersthilfsmittel kann insbesondere dazu ausgelegt sein, bei der Anwendung von erster Hilfe eingesetzt zu werden, wobei im Rahmen der vorliegenden Offenbarung neben der Versorgung einer verletzten Person auch eine Brandbekämpfung verstanden wird.

Bekannte Erste-Hilfe-Stationen müssen regelmäßig von geschultem Fachpersonal vor Ort auf ihre Einsatzfähigkeit kontrolliert werden. Hierbei werden insbesondere die Ersthilfsmittel einer Erste-Hilfe-Station auf Vollständigkeit kontrolliert. Wenn bei einer Kontrolle beispielsweise festgestellt wird, dass ein Verbandkasten einer Erste-Hilfe-Station fehlt, so kann dieser ersetzt werden. Derartige Kontrollen sind von Natur aus zeitintensiv. Auch kann es vorkommen, dass das Fachpersonal eine Erste-Hilfe-Station aus Versehen nicht kontrolliert.

Daher ist eine Technik wünschenswert, die diese Nachteile vermeidet. Insbesondere ist es ein Ziel der vorliegenden Erfindung, eine Erste-Hilfe-Station und ein Rechensystem bereitzustellen, die es ermöglichen, dass eine Erste-Hilfe-Station ohne zeitintensive Kontrollen immer einsatzbereit ist.

Diese Aufgabe ist durch eine Erste-Hilfe-Station gemäß Patentanspruch 1 und das Rechensystem gemäß Anspruch 12 gelöst.

Gemäß einem ersten Aspekt der Erfindung wird eine Erste-Hilfe-Station zum Erfassen und Melden einer Entnahme zumindest eines Ersthilfsmittels bereitgestellt. Die Erste-Hilfe-Station weist eine Halteeinrichtung zum Halten zumindest eines Ersthilfsmittels in der bzw. an der Erste-Hilfe-Station auf. Die Erste-Hilfe-Station weist ferner eine Überwachungseinrichtung zum Erfassen einer Entnahme des zumindest einen Ersthilfsmittels aus der bzw. von der Erste-Hilfe-Station auf. Außerdem weist die Erste-Hilfe-Station eine Kommunikationseinrichtung zum (z.B. selbsttätigen, autarken oder automatischen) Melden einer (z.B. der) erfassten Entnahme über eine digitale Kommunikationsverbindung an eine Überwachungsstelle auf.

Eine solche Erste-Hilfe-Station ermöglicht insbesondere, die Überwachungsstelle autark darüber zu informieren, ob ein Ersthilfsmittel aus bzw. von der Erste-Hilfe-Station entnommen worden ist. Eine lokale Kontrolle der Erste-Hilfe-Station durch geschultes Fachpersonal ist somit unnötig.

Die Überwachungsstelle kann ein Server sein oder einen Server umfassen. Die Überwachungsstelle kann eine personell besetzte Überwachungszentrale oder Leitzentrale sein. Die Überwachungsstelle kann ein Endgerät von Wartungspersonal sein oder umfassen.

Die digitale Kommunikationsverbindung kann eine (z.B. zumindest abschnittsweise) drahtlose Kommunikationsverbindung sein oder umfassen. Die digitale Kommunikationsverbindung kann eine Verbindung mit einem drahtlosen lokalen Netzwerk, WLAN, umfassen. Die digitale Kommunikationsverbindung kann eine Mobilfunkverbindung umfassen. Die digitale Kommunikationsverbindung kann eine (z.B. zumindest abschnittsweise) kabelgebundene Kommunikationsverbindung sein oder umfassen.

Die Halteeinrichtung kann eine lösbare Befestigung für ein Ersthilfsmittel umfassen. Die Halteeinrichtung kann eine Aufnahme oder einen Spender zum entnehmbaren Halten eines Ersthilfsmittels umfassen. Die Halteeinrichtung kann dazu ausgelegt sein, freistehend aufgestellt zu werden. Hierzu kann die Halteeinrichtung einen Standfuß umfassen. Alternativ kann die Halteeinrichtung dazu ausgelegt sein, an einer Wand oder Decke eines Gebäudes oder Fahrzeugs befestigt zu werden. Hierzu kann die Halteeinrichtung ein Befestigungsmittel und/oder eine Montageschnittstelle für eine Befestigungsmittel umfassen.

Die Überwachungseinrichtung kann zum Erfassen einer Entnahme des zumindest einen Ersthilfsmittels von der Halteeinrichtung eingerichtet sein. Die Überwachungseinrichtung kann dazu eingerichtet sein, die Entnahme jeder Art eines Ersthilfsmittels separat zu erfassen. Die Überwachungseinrichtung kann dazu eingerichtet sein, die Entnahme jedes einzelnen des zumindest einen Ersthilfsmittels separat zu erfassen. Die Überwachungseinrichtung kann dazu eingerichtet sein, die Entnahme einzelner Ersthilfsmittel derselben Art separat zu erfassen.

Die Überwachungseinrichtung kann einen oder mehrere Sensoren umfassen, beispielsweise einen Lichtsensor (z.B. zur Detektion von sichtbarem Licht oder Infrarotlicht), einen Magnetfeldsensor, einen Ultraschallsensor, einen Funksensor oder einen Radarsensor. Die Überwachungseinrichtung kann mehrere Sensoren unterschiedlicher Detektionstypen (z.B. optisch, akustisch, radioelektrisch oder magnetisch) umfassen. Jeweils ein Sensor der Überwachungseinrichtung kann einem Ersthilfsmittel zugeordnet sein. Alternativ ist zumindest ein Sensor der Überwachungseinrichtung mehreren Ersthilfsmitteln zugeordnet.

Das zumindest eine Ersthilfsmittel kann dazu ausgelegt sein, bei Durchführung einer Sofortmaßnahme eingesetzt zu werden, die insbesondere einer Lebensrettung und/oder Gesundheitserhaltung dient. Das zumindest eine Ersthilfsmittel kann dazu ausgelegt sein, bei einer Anwendung bzw. Leistung von erster Hilfe eingesetzt zu werden. Beispielsweise umfasst das zumindest eine Ersthilfsmittel zumindest ein Medizinprodukt. Beispielsweise umfasst das zumindest eine Ersthilfsmittel einen Verbandkasten, Verbandmaterial und/oder einen Defibrillator. Das Verbandmaterial kann ein Pflaster umfassen oder sein.

Die Erste-Hilfe-Station kann eine Benutzereingabeschnittstelle umfassen, die dazu eingerichtet ist, eine Benutzereingabe zu digitalisieren. Die Erste-Hilfe-Station kann dazu eingerichtet sein, die digitalisierte Benutzereingabe an ein digitales Verbandbuch weiterzugeben. Das digitale Verbandbuch kann in einem lokalen Speichermedium der Erste-Hilfe-Station gespeichert sein. Das digitale Verbandbuch kann auf einem über die digitale Kommunikationsverbindung erreichbaren Speichermedium gespeichert sein. Das digitale Verbandbuch kann auf einem Server gespeichert sein. Die Weitergabe der digitalisierten Benutzereingabe kann eine Aktualisierung des digitalen Verbandbuchs bewirken. Durch diese Ausgestaltung wird ein manuelles Einsammeln papierbasierter Formulare eines Verbandbuchs hinfällig. Dies kann zu weiterer Zeitersparnis führen und dient ferner dem Datenschutz sensibler personenbezogener Gesundheitsdaten, die bei papierbasierten Formularen meist frei einsehbar sind.

Die Kommunikationseinrichtung kann dazu eingerichtet sein, Betriebsanweisungen oder/und Sicherheitsanweisungen zu empfangen (z.B. über die digitale Kommunikationsverbindung oder die Benutzereingabeschnittstelle). Die Erste-Hilfe-Station kann eine Anzeigeeinrichtung umfassen. Beispielsweise ist die Anzeigeeinrichtung dazu ausgelegt, die Betriebsanweisungen oder/und Sicherheitsanweisungen anzuzeigen. So können die angezeigten Betriebs- und/oder Sicherheitsanweisungen auf zeitsparende und sichere Weise aktualisiert werden. Beispielsweise umfasst die Anzeigeeinrichtung einen berührungsempfindlichen Bildschirm. Die Benutzereingabeschnittstelle kann die Anzeigeeinrichtung, insbesondere den berührungsempfindlichen Bildschirm, umfassen. Alternativ kann die Anzeigeeinrichtung die Benutzereingabeschnittstelle umfassen.

Die Überwachungseinrichtung kann einen Lichtsensor umfassen. Die Überwachungseinrichtung kann dazu eingerichtet sein, eine Entnahme (z.B. von Verbandmaterial) zu erfassen, sobald oder falls von dem Lichtsensor detektiertes Licht eine vorbestimmte Eigenschaft aufweist. Die vorbestimmte Eigenschaft kann eine (z.B. über einen vorbestimmten Mindestzeitraum detektierte) vorbestimmte Lichtintensität, ein (z.B. über einen vorbestimmten Mindestzeitraum detektiertes) Überschreiten oder Unterschreiten einer vorbestimmten Lichtintensitätsschwelle, oder ein (z.B. über einen vorbestimmten Mindestzeitraum detektiertes) vorbestimmtes Lichtspektrum sein. Die Überwachungseinrichtung kann dazu eingerichtet sein, eine Entnahme einzelner Pflaster zu erfassen.

Beispielsweise ist der Lichtsensor als Teil einer Lichtschranke der Überwachungseinrichtung ausgebildet. Die Lichtschranke kann so angeordnet sein, dass sie bei einer Entnahme (z.B. von Verbandmaterial oder von einem Pflaster) durchbrochen wird. Die Überwachungseinrichtung kann dazu eingerichtet sein, eine Entnahme zu erfassen, sobald oder falls von dem Lichtsensor detektiertes Licht ein Durchbrechen der Lichtschranke indiziert, beispielsweise sobald von dem Lichtsensor detektiertes Licht unter eine vorbestimmte Lichtintensitätsschwelle absinkt.

Das zumindest eine Ersthilfsmittel kann zumindest ein Brandbekämpfungsmittel umfassen, z. B. zumindest eine Feuerlöschvorrichtung. Das zumindest eine Ersthilfsmittel kann insbesondere einen Feuerlöscher und/oder eine Feuerlöschdecke umfassen. Beispielsweise umfasst das zumindest eine Ersthilfsmittel ein oder mehrere Medizinprodukte und zumindest eine Feuerlöschvorrichtung.

Die Überwachungseinrichtung kann einen Magnetfeldsensor umfassen. Die Überwachungseinrichtung kann dazu eingerichtet sein, eine Entnahme (z.B. eines Verbandkastens oder eines Feuerlöschers) zu erfassen, sobald ein von dem Magnetfeldsensor detektiertes Magnetfeld eine vorbestimmte Eigenschaft aufweist. Die vorbestimmte Eigenschaft kann eine (z.B. über einen vorbestimmten Mindestzeitraum detektierte) vorbestimmte Magnetfeldintensität, ein (z.B. über einen vorbestimmten Mindestzeitraum detektiertes) Überschreiten oder Unterschreiten einer vorbestimmten Magnetfeldstärke, oder eine (z.B. über einen vorbestimmten Mindestzeitraum detektierte) Magnetfeldrichtung sein. Der Magnetfeldsensor kann als Sensor eines Magnet-Sensor-Paares ausgebildet sein. Der Magnet des Magnet-Sensor-Paares kann an dem Ersthilfsmittel angeordnet sein, dessen Entnahme mittels des Magnetfeldsensors des Magnet-Sensor-Paares ermittelt werden soll.

Anstelle des Magnetfeldsensors kann ein induktiver Sensor vorgesehen sein. In diesem Fall kann anstelle oder zusätzlich zu dem Magnet ein leitfähiges oder/und ferromagnetisches Element vorgesehen sein. Dieses Element kann Teil des Ersthilfsmittels sein (z.B. ein Teil des Gehäuses des Verbandkastens oder des Feuerlöschers).

Die Überwachungseinrichtung kann einen Funksensor umfassen. Die Überwachungseinrichtung kann dazu eingerichtet sein, eine Entnahme (z.B. eines Verbandkastens oder eines Feuerlöschers) zu erfassen, sobald ein von dem Funksensor detektiertes Funksignal eine vorbestimmte Eigenschaft aufweist. Die vorbestimmte Eigenschaft kann eine (z.B. über einen vorbestimmten Mindestzeitraum detektierte) vorbestimmte Funksignalintensität, ein (z.B. über einen vorbestimmten Mindestzeitraum detektiertes) Überschreiten oder Unterschreiten einer vorbestimmten Funksignalstärke, oder eine (z.B. über einen vorbestimmten Mindestzeitraum detektierte) Funksignalrichtung sein. Hierbei kann das Funksignal spezifisch für das Ersthilfsmittel sein, dessen Entnahme mittels des Funksensors überwacht wird. Der Funksensor kann als Empfänger eines Empfänger-Sender-Paares ausgebildet sein. Der Sender des Empfänger-Sender-Paares kann an dem Ersthilfsmittel angeordnet sein, dessen Entnahme mittels des Funkempfängers ermittelt werden soll. Der Sender kann ein senderspezifisches Funksignal aussenden. Das von dem Sender ausgesendete Signal kann Informationen über den Sender oder/und das dem Sender zugeordnete Ersthilfsmittel transportieren. Die Informationen können einen Typ, eine Artikelnummer, ein Mindesthaltbarkeitsdatum oder/und eine Bezeichnung des dem Sender zugeordneten Ersthilfsmittels umfassen. Die Überwachungseinrichtung kann dazu ausgelegt sein, mittels eines Funkempfängers Funksignale einer Vielzahl von jeweils einem Ersthilfsmittel zugeordneten Funksendern zu empfangen. In diesem Fall kann eine Entnahme mehrerer Ersthilfsmittel unter Verwendung eines einzigen Funkempfängers erfasst werden.

Der Funksensor bzw. Funkempfänger kann ein Radiofrequenzidentifikations- (RFID-) Sensor, ein Nahfeldkommunikations- (NFC-) Sensor, oder ein Bluetooth-Sensor sein. Derartige Sensoren können als Funkempfänger bezeichnet werden. Ein einem Ersthilfsmittel zugeordneter Funksender kann an diesem Ersthilfsmittel angeordnet, insbesondere befestigt sein. Der Funkempfänger und der Funksender können für eine Nachfeldkommunikation eingerichtet sein.

Die Überwachungseinrichtung kann dazu eingerichtet sein, ein Eintreten oder Hineingreifen einer Person in einen dem zumindest einen Ersthilfsmittel benachbarten Raumbereich zu detektieren. Die Überwachungseinrichtung kann alternativ oder zusätzlich dazu ausgelegt sein, eine Präsenz eines Objekts oder einer Person in einem dem zumindest einen Ersthilfsmittel benachbarten Raumbereich zu detektieren. Der Raumbereich ist beispielsweise ein Raumbereich, in welchen eine Person zur Entnahme des zumindest einen Ersthilfsmittels eintreten oder hineingreifen muss. Der Raumbereich kann auf der Seite der Halteeinrichtung liegen, auf welcher das zumindest eine Ersthilfsmittel angeordnet ist. Der Raumbereich kann von einem Boden vor der Erste-Hilfe-Station begrenzt sein, auf welchem eine Person stehen muss, um das zumindest eine Ersthilfsmittel zu entnehmen.

Die Überwachungseinrichtung kann ein Abstandsmesssystem umfassen, das zum Messen eines Abstands eines Objekts oder einer Person von der Erste-Hilfe-Station und/oder von dem zumindest einen Ersthilfsmittel eingerichtet ist. Das Abstandsmesssystem kann dazu eingerichtet sein, den Abstand mittels Laufzeitmessung eines ausgesandten optischen oder akustischen Referenzsignals zu ermitteln. Das Abstandsmesssystem kann dazu eingerichtet sein, ein Unterschreiten eines vorbestimmten Mindestabstands zu detektieren. Die Überwachungseinrichtung kann dazu ausgelegt sein, das Eintreten oder Hineingreifen in den Raumbereich zu detektieren, sobald oder falls das Abstandsmesssystem das Unterschreiten des Mindestabstands detektiert. Die Überwachungseinrichtung kann dazu ausgelegt sein, eine Präsenz eines Objekts oder einer Person in dem Raumbereich zu detektieren, sobald oder falls das Abstandsmesssystem das Unterschreiten des Mindestabstands detektiert. Die Kommunikationseinrichtung kann ferner dazu eingerichtet sein, das detektierte Eintreten oder Hineingreifen zu melden (z.B. über die digitale Kommunikationsverbindung und insbesondere an die Überwachungsstelle). Die Kommunikationseinrichtung kann ferner dazu eingerichtet sein, die detektierte Präsenz zu melden (z.B. über die digitale Kommunikationsverbindung und insbesondere an die Überwachungsstelle).

Beispielsweise kann die Überwachungseinrichtung dazu eingerichtet sein, eine Entnahme unter der Bedingung zu erfassen, dass zuvor ein Eintreten oder Hineingreifen in den Raumbereich detektiert wurde. So ist denkbar, dass einer oder mehrere Sensoren der Überwachungseinrichtung, die dem Erfassen einer Entnahme eines Ersthilfsmittels dienen, erst dann aktiviert oder ausgelesen werden, wenn ein Eintreten oder Hineingreifen in den Raumbereich detektiert wurde. Zusätzlich oder alternativ kann die Kommunikationseinrichtung dazu eingerichtet sein, die erfasste Entnahme unter der Bedingung zu melden, dass in einer vorgegebenen zeitlichen Nähe zu der erfassten Entnahme ein Eintreten oder Hineingreifen in den Raumbereich detektiert wurde. Dies kann die Häufigkeit falschpositiver Erfassungen bzw. Fehlmeldungen von Entnahmen reduzieren.

Die Erste-Hilfe-Station kann dazu eingerichtet sein, bei Vorliegen eines eine benötigte Wartung der Erste-Hilfe-Station indizierenden Alarmereignisses einen Alarm auszulösen oder auszugeben. Ein solches Alarmereignis liegt insbesondere dann vor, wenn die Überwachungseinrichtung eine Entnahme erfasst hat, eine Anzahl von durch die Überwachungseinrichtung erfassten Entnahmen (z.B. eines des zumindest einen Ersthilfsmittels) einen vorbestimmten (z.B. Ersthilfsmittel-spezifischen) Grenzwert überschreitet, ein Haltbarkeitsdatum eines (z.B. des zumindest einen) Ersthilfsmittels überschritten ist, mittels eines Beschleunigungssensors der Erste-Hilfe-Station eine Erschütterung detektiert wird, die einen vorbestimmten Grenzwert überschreitet, ein Füllstand eines Energiespeichers der Erste-Hilfe-Einrichtung unter einen vorbestimmten Schwellwert fällt, ein Wartungstermin vorliegt und/oder die Erste-Hilfe-Station eine Meldung über ein Vorliegen des Alarmereignisses empfangen hat (z.B. über die Kommunikationseinrichtung oder die Benutzereingabeschnittstelle). Die Erste-Hilfe-Station kann dazu eingerichtet sein, den Alarm über eine Alarmeinrichtung (z.B. einen Lautsprecher, eine Sirene, eine Warnleuchte oder die Anzeigeeinrichtung) der Erste-Hilfe-Station auszugeben, den Alarm eines (z.B. mit der Erste-Hilfe-Station gekoppelten) Hausalarmsystems auszulösen oder/und den Alarm über die digitale Kommunikationsverbindung auszulösen.

Die Kommunikationseinrichtung kann ferner dazu ausgeführt sein, mittels Nahfeldkommunikation ein Identifikationsgerät zu erkennen und basierend auf dem Erkennen eine Änderung einer Konfiguration der Erste-Hilfe-Station zu bewirken. Das Identifikationsgerät kann einen Nahfeldkommunikations-Sender oder einen Nahfeldkommunikations-Transponder umfassen. Das Identifikationsgerät kann einen Radiofrequenz-Transponder umfassen. Das Identifikationsgerät ist beispielsweise keinem Ersthilfsmittel zugeordnet, sondern einer autorisierten Person wie z.B. Wartungspersonal. Die Änderung der Konfiguration der Erste-Hilfe-Station kann ein Freischalten einer ansonsten gesperrten Funktion der Erste-Hilfe-Station, der Benutzereingabeschnittstelle und/oder der Anzeigeeinrichtung umfassen. Die Änderung der Konfiguration der Erste-Hilfe-Station kann ein Zurücksetzen eines Zählers der Erste-Hilfe-Station umfassen. Die Änderung der Konfiguration der Erste-Hilfe-Station kann ein Zurücksetzen eines Alarms oder ein Löschen eines (z.B. Vorliegens eines) Alarmereignisses umfassen. Beispielsweise kann der Zähler oder Alarm nur dann zurückgesetzt werden, wenn das entnommene Ersthilfsmittel ersetzt worden ist. Die Überwachungseinrichtung kann zum Erfassen des Ersetzens des entnommenen Ersthilfsmittels ausgelegt sein. Beispielsweise wird ein Zähler oder Alarm nur dann zurückgesetzt bzw. ein Alarmereignis nur dann gelöscht, wenn über die Benutzereingabeschnittstelle, insbesondere nach dem Freischalten einer Rücksetzungs-Funktion der Benutzereingabeschnittstelle, eine entsprechende Eingabe erfasst wird.

Gemäß einem zweiten Aspekt der Erfindung wird ein Rechensystem zum Verarbeiten von Meldungen der Erste-Hilfe-Station gemäß dem ersten Aspekt bereitgestellt. Das Rechensystem umfasst eine Kommunikationsschnittstelle für die digitale Kommunikationsverbindung und ist dazu eingerichtet, von der Erste-Hilfe-Station über die Kommunikationsschnittelle eine Meldung einer erfassten Entnahme des zumindest einen Ersthilfsmittels zu empfangen und basierend auf der Meldung der erfassten Entnahme eine vorbestimmte Aktion durchzuführen.

Das Rechensystem kann dazu eingerichtet sein, von der Erste-Hilfe-Station über die Kommunikationsschnittelle eine Meldung (i) eines Eintretens oder Hineingreifens einer Person in einen dem zumindest einen Ersthilfsmittel benachbarten Raumbereich oder (ii) einer Präsenz eines Objekts in einem dem zumindest einen Ersthilfsmittel benachbarten Raumbereich zu empfangen, und die vorbestimmte Aktion ferner basierend auf der Meldung des Eintretens oder Hineingreifens durchzuführen. Bezüglich des Raumbereichs und der Detektion der Präsenz, des Eintretens oder Hineingreifens durch die Erste-Hilfe-Station gelten die obenstehenden Ausführungen zum ersten Aspekt entsprechend.

Die vorbestimmte Aktion kann ein Bestimmen umfassen, ob ein Alarmereignis vorliegt, welches eine benötigte Wartung der Erste-Hilfe-Station indiziert. Das Rechensystem kann dazu eingerichtet sein, bei Vorliegen des Alarmereignisses einen Alarm auszulösen, beispielsweise über die Erste-Hilfe-Station. Das Rechensystem kann dazu eingerichtet sein, bei Vorliegen des Alarmereignisses eine Meldung über das Vorliegen des Alarmereignisses zu senden, beispielsweise an die Erste-Hilfe-Station, an eine Leitzentrale und/oder an ein Endgerät von Wartungspersonal. Das Rechensystem kann dazu ausgelegt sein, die Meldung über das Vorliegen des Alarmereignisses an die Erste-Hilfe-Station zu senden, falls oder sobald das Rechensystem eine Benutzerbestätigung des Alarmereignisses empfängt (z.B. von einem mit dem Rechensystem kommunikativ verbundenen Endgerät). Bezüglich des Vorliegens des Alarmereignisses gelten die obenstehenden Ausführungen zum ersten Aspekt entsprechend.

Die vorbestimmte Aktion kann alternativ oder zusätzlich ein Erstellen einer Anzeige basierend auf der empfangenen Meldung bzw. den empfangenen Meldungen umfassen. Das Rechensystem kann dazu eingerichtet sein, die Anzeige so zu erstellen, dass sie einen Benutzer über einen aktuellen Zustand oder eine Einsatzfähigkeit des Erste-Hilfe-Systems informiert. Die erstellte Anzeige kann (z.B. alle) Informationen aus der bzw. den von der Erste-Hilfe-Station empfangenen Meldungen visualisieren. Das Rechensystem kann dazu eingerichtet sein, die Anzeige auf einem Ausgabebildschirm auszugeben. Der Ausgabebildschirm kann Teil des Rechensystems oder Teil eines mit dem Rechensystem kommunikativ verbundenen Endgeräts (z.B. der Überwachungsstelle) sein.

Das Rechensystem kann dazu eingerichtet sein, Betriebsanweisungen und/oder Sicherheitsanweisungen an die Erste-Hilfe-Station zu senden. Das Rechensystem kann dazu eingerichtet sein, die zu sendenden Betriebsanweisungen und/oder Sicherheitsanweisungen von einem Server abzurufen, sobald diese aktualisiert worden sind. Das Rechensystem kann dazu eingerichtet sein, von der Erste-Hilfe-Station eine digitalisierte Benutzereingabe zu empfangen und basierend auf der digitalisierten Benutzereingabe ein digitales Verbandbuch zu aktualisieren. Das digitale Verbandbuch kann auf einem Speichermedium des Rechensystems gespeichert sein.

Gemäß einem dritten Aspekt der Erfindung wird ein Gesamtsystem bereitgestellt, das eine Erste-Hilfe-Station gemäß dem ersten Aspekt und ferner zumindest ein Ersthilfsmittel (z.B. das von der Halteeinrichtung gehaltene zumindest eine Ersthilfsmittel) und/oder das Rechensystem gemäß dem zweiten Aspekt umfasst. Das Gesamtsystem kann sämtliche Ersthilfsmittel der Erste-Hilfe-Station umfassen.

Ein Ausführungsbeispiel der vorliegenden Erfindung wird im Folgenden anhand der beigefügten, schematischen Figur näher beschrieben. Es zeigt:

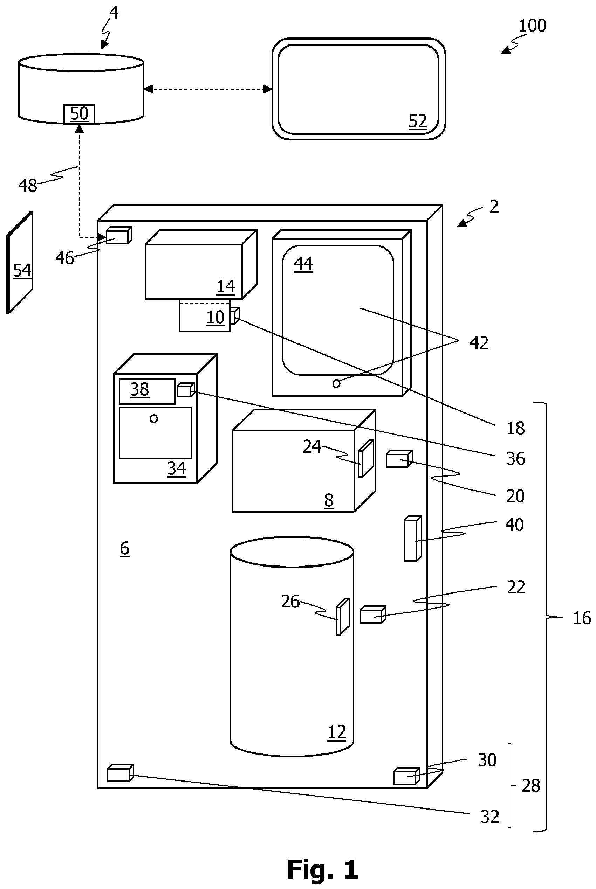

- Fig. 1: eine schematische Darstellung eines Ausführungsbeispiels eines Gesamtsystems gemäß der vorliegenden Erfindung.

Fig. 1 zeigt eine schematische Darstellung eines Ausführungsbeispiels eines Gesamtsystems 100 gemäß der vorliegenden Erfindung. Das Gesamtsystem 100 umfasst eine Erste-Hilfe-Station 2 und ein Rechensystem 4.

Die Erste-Hilfe-Station 2 umfasst eine Halteeinrichtung 6 zum Halten zumindest eines Ersthilfsmittels in der bzw. an der Erste-Hilfe-Station 2. Die Halteeinrichtung 6 kann so ausgelegt sein, dass sie an einer Wand oder Decke eines Gebäudes anbringbar ist. Alternativ kann die Halteeinrichtung 6 einen Standfuß umfassen, sodass die Erste-Hilfe-Station 2 freistehend platziert werden kann. Die Erste-Hilfe-Station 2 kann akkubetrieben oder netzbetrieben sein. Beispielsweise umfasst die Erste-Hilfe-Station 2 eine Energiespeichervorrichtung, deren Energielevel bzw. Füllstand sie überwacht.

Das zumindest eine Ersthilfsmittel ist dazu ausgelegt, bei der Anwendung von erster Hilfe eingesetzt zu werden. In dem gezeigten Ausführungsbeispiel hält die Halteeinrichtung 6 als Ersthilfsmittel einen Verbandkasten 8, Verbandmaterial 10 und einen Feuerlöscher 12. Die Halteeinrichtung 6 umfasst hierzu einen Spender 14, in welchem das Verbandmaterial 10 in Form eines Pflasterstreifens gehalten wird. Der Pflasterstreifen kann aus dem Spender 14 herausgezogen und abschnittsweise abgetrennt werden. So lassen sich dem Spender 14 als Ersthilfsmittel einzelne Pflaster entnehmen. Der Verbandkasten 8 sowie der Feuerlöscher 12 können jeweils von der Halteeinrichtung 6 entkoppelt und als Ganzes entfernt bzw. entnommen werden. Es können zusätzliche oder alternative Ersthilfsmittel von der Halteeinrichtung 6 gehalten werden, beispielsweise ein Defibrillator und/oder eine Feuerlöschdecke .

Die Erste-Hilfe-Station 2 umfasst ferner eine Überwachungseinrichtung 16. Die Überwachungseinrichtung 16 ist dazu eingerichtet, eine Entnahme des jeweiligen Ersthilfsmittels zu erfassen. Insbesondere dient die Überwachungseinrichtung 16 dem separaten Erfassen einer Entnahme einzelner Pflaster des Verbandmaterials 10, einer Entnahme des Verbandkastens 8 und einer Entnahme des Feuerlöschers 12.

Die Überwachungseinrichtung 16 umfasst einen Lichtsensor 18, der einen Teil einer Lichtschranke bildet. Bei der Entnahme von Verbandmaterial aus dem Spender 14 wird die Lichtschranke durchbrochen und die von dem Lichtsensor 18 detektierte Lichtintensität sinkt unter einen vorbestimmten Grenzwert ab. Die Überwachungseinrichtung 16 erfasst daraufhin die Entnahme von Verbandmaterial.

Die Überwachungseinrichtung 16 umfasst ferner einen ersten Magnetfeldsensor 20 und einen zweiten Magnetfeldsensor 22. Der erste Magnetfeldsensor 20 ist dazu ausgelegt, ein Magnetfeld zu detektieren, welches von einem ersten Magneten 24 ausgeht. Der erste Magnet 24 ist an dem Verbandkasten 8 befestigt. Bei einer Entnahme des Verbandkastens 8 von der Halteeinrichtung 6 entfernt sich der erste Magnet 24 von dem ersten Magnetfeldsensor 20. Dies bewirkt ein Absinken der von dem ersten Magnetfeldsensor 20 detektierten Magnetfeldintensität. Sobald das von dem ersten Magnetsensor 20 detektierte Magnetfeld unter einen vorbestimmten Schwellenwert abfällt, erfasst die Überwachungseinrichtung 16 die Entnahme des Verbandkastens 8 aus der Erste-Hilfe-Station 2.

Der zweite Magnetfeldsensor 22 dient der Detektion eines Magnetfelds, welches von einem zweiten Magneten 26 ausgeht. Der zweite Magnet 26 ist an dem Feuerlöscher 12 befestigt. Sobald die von dem zweiten Magnetfeldsensor 22 detektierte Magnetfeldintensität unter einen vorbestimmten Grenzwert absinkt, erfasst die Überwachungseinrichtung 16 eine Entnahme des Feuerlöschers 12 aus der Erste-Hilfe-Station 2. Die hier beschriebenen Magnetfeldsensoren können sogenannte Reed-Kontakte oder Hall-Sensoren sein.

Anstelle der oder zusätzlich zu den hier genannten Sensortypen kann die Überwachungseinrichtung 16 zum Erfassen einer Entnahme eines Ersthilfsmittels auch andere Sensoren oder Erfassungseinrichtungen umfassen, beispielsweise einen Ultraschallsensor, einen Radarsensor, einen Funksensor bzw. Funkempfänger, einen Beschleunigungssensor, einen Kapazitätssensor, einen Bewegungsmelder, einen Schaltkontakt und/oder eine Kamera.

Die Überwachungseinrichtung 16 umfasst in dem dargestellten Ausführungsbeispiel ferner ein Abstandsmesssystem 28. Das Abstandsmesssystem 28 weist eine erste Abstandsmesseinrichtung 30 und eine zweite Abstandsmesseinrichtung 32 auf. Die Abstandsmesseinrichtungen 30, 32 sind an einem unteren, dem Boden bzw. Standfuß zugewandten Ende der Halteeinrichtung 6 angeordnet. Die Abstandsmesseinrichtungen 30, 32 erfassen einen Raumbereich vor der Erste-Hilfe-Station 2. Beispielsweise ist der Raumbereich so gewählt, dass eine Person in den Raumbereich eintreten muss, um das Verbandmaterial 10, den Verbandkasten 8 oder den Feuerlöscher 12 aus der Erste-Hilfe-Station 2 zu entnehmen. Einerseits ermöglicht dies, festzustellen, ob eine Person den Raumbereich betreten hat, um ein Ersthilfsmittel zu entnehmen. Andererseits kann so festgestellt werden, ob in dem freizuhaltenden Raumbereich ein Objekt abgestellt wurde, welches den freien Zugang zur Erste-Hilfe-Station blockiert.

Die Abstandsmesseinrichtungen 30, 32 können jeweils dazu eingerichtet sein, anhand einer Laufzeitmessung eines akustischen oder optischen Referenzsignals (z.B. Lidarbasiert) einen Abstand zu einem Objekt oder einer Person von der jeweiligen Abstandsmesseinrichtung zu ermitteln. Die Überwachungseinrichtung 16 kann dazu ausgelegt sein, anhand der von den Abstandsmesseinrichtungen 30, 32 ermittelten Abstände, insbesondere durch Triangulation, einen Ort des Objekts oder der Person zu ermitteln. Die Überwachungseinrichtung 16 kann dazu eingerichtet sein, basierend auf dem ermittelten Abstand oder den ermittelten Abständen, insbesondere basierend auf dem ermittelten Ort der Person, ein Eintreten einer Person in den Raumbereich vor der Erste-Hilfe-Station 2 zu detektieren oder/und eine Präsenz eines Objekts in dem Raumbereich zu detektieren.

Alternativ oder zusätzlich zu dem beschriebenen Abstandsmesssystem 28 kann eine Abstandsmesseinrichtung vorgesehen sein, die dazu ausgelegt ist, ein Hineingreifen einer Person in einen Raumbereich zu detektieren, in den die Person zur Entnahme eines Ersthilfsmittels hineingreifen muss, oder/und eine Präsenz eines Objekts in dem Raumbereich zu detektieren. Zur Detektion des Eintretens bzw. Hineingreifens oder einer Präsenz des Objekts muss nicht zwangsläufig eine Abstandsmesseinrichtung vorhanden sein - es kann genügen, eine Detektionsvorrichtung (z.B. einen Bewegungsmelder mit einem vorbestimmten Sichtbereich) bereitzustellen, die (z.B. binär) detektiert, ob sich ein Objekt oder eine Person in den überwachten Raumbereich bzw. in den Sichtbereich der Detektionsvorrichtung hineinbewegt hat.

Die Erste-Hilfe-Station 2 kann einen Briefkasten 34 und eine Detektionseinrichtung 36 zum Erfassen eines Einwurfs in den Briefkasten 34 umfassen. Die Detektionseinrichtung 36 kann dazu ausgelegt sein, eine Bewegung einer Einwurfklappe 38 des Briefkasten 34 zu detektieren, um dadurch einen Einwurf in den Briefkasten 34 zu erfassen. Die Detektionseinrichtung 36 kann hierzu einen Magnetfeldsensor umfassen, der zur Detektion eines an der Einwurfklappe 38 befestigten Magneten angeordnet ist. Die Detektionseinrichtung 36 kann Teil der Überwachungseinrichtung 16 sein.

Die Überwachungseinrichtung 16 kann ferner eine Atmosphärenmesseinrichtung 40 umfassen. Die Atmosphärenmesseinrichtung 40 kann dazu ausgelegt sein, Rauch zu detektieren, eine Konzentration von gesundheitsrelevantem Gas (z.B. Sauerstoff, Kohlenstoffdioxid oder Kohlenstoffmonoxid) zu messen, eine Umgebungstemperatur zu messen, eine Luftfeuchtigkeit zu messen und/oder einen Umgebungsluftdruck zu messen.

In dem dargestellten Ausführungsbeispiel umfasst die Erste-Hilfe-Station 2 ferner eine Benutzereingabeschnittstelle 42. Diese ist dazu ausgelegt, eine Benutzereingabe zu digitalisieren. Die Erste-Hilfe-Station 2 ist dazu eingerichtet, die digitalisierte Benutzereingabe anschließend an ein digitales Verbandbuch weiterzugeben, welches entweder lokal auf einem Speichermedium der Erste-Hilfe-Hilfestation 2 oder auf einem separaten Speichermedium gespeichert ist. Beispielsweise ist das digitale Verbandbuch auf einem Server gespeichert, insbesondere auf einem Speichermedium des Rechensystems 4.

Die Erste-Hilfe-Station 2 umfasst ferner eine Anzeigeeinrichtung 44. Die Anzeigeeinrichtung 44 dient der Anzeige von Betriebs- und/oder Sicherheitsinformationen. Die Anzeigeeinrichtung 44 kann einen berührungsempfindlichen Bildschirm umfassen. In dem dargestellten Ausführungsbeispiel umfasst die Benutzereingabeschnittstelle 42 den berührungsempfindlichen Bildschirm der Anzeigeeinrichtung 44 sowie eine Eingabetaste (z.B. zum Aktivieren des Bildschirms oder zum Bestätigen einer Benutzereingabe).

Die Erste-Hilfe-Station 2 umfasst ferner eine Kommunikationseinrichtung 46. Die Kommunikationseinrichtung 46 ist dazu ausgelegt, eine erfasste Entnahme über eine digitale Kommunikationsverbindung 48 an eine Überwachungsstelle zu melden. Bei der Überwachungsstelle kann es sich um einen Server, eine Leitzentrale, ein personell besetztes Büro oder um ein Endgerät (z.B. ein Mobiltelefon, ein Computer oder ein tragbares Alarmgerät) von Wartungspersonal handeln. Das Rechensystem 4 kann ein Server und/oder Teil der Überwachungsstelle sein. Die digitale Kommunikationsverbindung 48 umfasst eine zumindest abschnittsweise drahtlose Kommunikationsverbindung, beispielsweise eine drahtlose lokale Netzwerkverbindung oder eine Mobilfunkverbindung.

Die Kommunikationseinrichtung 46 ist dazu eingerichtet, an die Überwachungsstelle (z. B. alle) mittels der Überwachungseinrichtung 16 erfasste Vorkommnisse bzw. Messdaten selbsttätig zu melden, beispielsweise eine erfasste Entnahme eines Ersthilfsmittels, eine Anzahl erfasster Entnahmen eines Ersthilfsmittels, einen detektierten Abstand eines Objekts oder einer Person (z.B. von der Erste-Hilfe-Station 2, von dem Abstandsmesssystem 28 oder von einem Ersthilfsmittel an bzw. in der Erste-Hilfe-Station 2), ein detektiertes Eintreten oder Eingreifen in den Raumbereich durch eine Person, eine detektierte Präsenz eines Objekts in dem Raumbereich, einen erfassten Einwurf in den Briefkasten 34, eine Detektion von Rauch, eine gemessene Gaskonzentration, eine gemessene Umgebungstemperatur, eine gemessene Luftfeuchtigkeit und/oder einen gemessenen Umgebungsluftdruck. Die Kommunikationseinrichtung kann an die Überwachungsstelle die digitalisierte Benutzereingabe selbsttätig melden oder weiterleiten.

Das Rechensystem 4 umfasst eine Kommunikationsschnittstelle 50 für die digitale Kommunikationsverbindung 48 und ist dazu eingerichtet, eine oder mehrere Meldungen, insbesondere über die (z. B. alle) erfassten Vorkommnisse bzw. Messdaten und/oder die digitalisierte Benutzereingabe, von der Erste-Hilfe-Station 2 zu empfangen. Das Rechensystem 4 ist dazu ausgelegt, basierend auf der empfangenen Meldung bzw. den empfangenen Meldungen zumindest eine vorbestimmte Aktion durchzuführen. Das Rechensystem 4 kann Teil einer Rechnerwolke bzw. Cloud sein.

Eine vorbestimmte Aktion umfasst ein Generieren oder Erzeugen einer Anzeige. Diesbezüglich ist das Rechensystem 4 dazu eingerichtet, basierend auf der bzw. den empfangenen Meldungen eine Anzeige zu generieren, die einen Benutzer über einen aktuellen Status der Erste-Hilfe-Station 2 und/oder eine Änderung desselben informiert. Insbesondere kann die Anzeige so generiert werden, dass sie einen Benutzer über eine erfasste Entnahme eines Ersthilfsmittels der Erste-Hilfe-Station 2 informiert. Das Rechensystem 4 ist dazu ausgelegt, die generierte Anzeige auf einem Ausgabebildschirm 52 auszugeben. Der Ausgabebildschirm 52 kann Teil des Rechensystems 4 sein oder mit diesem kommunikativ in Verbindung stehen. Die erzeugte Anzeige kann ein sogenanntes Dashboard umfassen.

Eine andere vorbestimmte Aktion umfasst ein Feststellen eines Vorliegens eines Alarmereignisses, welches eine benötigte Wartung der Erste-Hilfe-Station 2 indiziert. Das Rechnersystem 4 ist dazu eingerichtet, basierend auf der bzw. den empfangenen Meldungen zu bestimmen, ob ein Alarmereignis vorliegt, welches eine benötigte Wartung der Erste-Hilfe-Station 2 indiziert. Das Rechensystem 4 kann dazu ausgelegt sein, verschiedene Typen von Alarmereignissen zu bestimmen, die für eine Einsatzbereitschaft der Erste-Hilfe-Station 2 unterschiedlich kritisch sind.

Ein besonders kritisches Alarmereignis liegt beispielsweise dann vor, wenn (z.B. innerhalb eines vorbestimmten Zeitraums nach der Detektion des Eintretens) eine vollständige Entnahme des Ersthilfsmittels (z.B. des Verbandkastens 8 oder des Feuerlöschers 12) erfasst wurde. Ein besonders kritisches Alarmereignis liegt beispielsweise auch dann vor, wenn detektiert wurde, dass eine Anzahl erfasster Entnahmen des Ersthilfsmittels (z.B. des Verbandmaterials 10) einen vorbestimmten Grenzwert überschreitet. Ein kritisches Alarmereignis kann vorliegen, wenn eine Präsenz eines Objekts in dem Raumbereich vor der Erste-Hilfe-Station 2 detektiert wurde. Ein weniger kritisches Alarmereignis kann beispielsweise vorliegen, wenn die Anzahl erfasster Entnahmen des Ersthilfsmittels (z.B. des Verbandmaterials 10) den vorbestimmten Grenzwert nicht überschreitet. Ein besonders kritisches Alarmereignis kann dann vorliegen, wenn ein Haltbarkeitsdatum eines Ersthilfsmittels überschritten ist. Ein weniger kritisches Alarmereignis kann vorliegen, wenn eine Eingabe über die Benutzereingabeschnittstelle 42 erfasst wurde, die an das digitale Verbandbuch weitergeleitet wurde oder weiterzuleiten ist.

Das Rechensystem 4 ist dazu ausgelegt, nach einer Feststellung, dass ein Alarmereignis vorliegt, eine entsprechende Meldung in die generierte oder zu generierende Anzeige einzufügen. Anhand der Anzeige kann ein Nutzer entscheiden, ob das bestimmte Alarmereignis basierend auf den von dem Rechensystem 4 empfangenen Daten bzw. Meldungen korrekt ermittelt wurde. Der Nutzer kann das festgestellte Alarmereignis daraufhin manuell bestätigen. Das Rechensystem 4 ist dazu ausgelegt, eine Meldung über die manuelle Bestätigung zu empfangen, und nach dem Empfangen der Bestätigung das Vorliegen des Alarmereignisses über die digitale Kommunikationsverbindung 48 an die Erste-Hilfe-Station 2 zu melden. Die Erste-Hilfe-Station ist dazu eingerichtet, beim Empfangen der Meldung über das Vorliegen des Alarmereignisses einen Alarm auszulösen. Der Alarm wird beispielsweise nur im Fall eines besonders kritischen Alarmereignisses ausgelöst. Je nach Typ des vorliegenden Alarmereignisses kann ein anderer Alarm ausgelöst werden. Alternativ oder zusätzlich kann das Rechensystem 4 oder die Erste-Hilfe-Station bei Vorliegen eines Alarmereignisses eine (z.B. SMS- oder E-Mail-) Nachricht an mit der Wartung der Erste-Hilfe-Station 2 betrautes Wartungspersonal versenden.

Die manuelle Bestätigung eines von dem Rechensystem 4 festgestellten Alarmereignisses ist nicht unbedingt notwendig. Die Erste-Hilfe-Station 2 könnte auch direkt über das von dem Rechensystem 4 bestimmte Alarmereignis informiert werden, ohne dass es eines personellen Eingreifens bedürfte. Es ist auch denkbar, dass das Vorliegen eines Alarmereignisses nicht durch das Rechensystem 4, sondern lokal auf der Erste-Hilfe-Station 2 bestimmt wird. Hierbei kann es genügen, wenn die Erste-Hilfe-Station 2 die Überwachungsstelle über das Vorliegen des Alarmereignisses, welches durch eine erfasste Entnahme ausgelöst wird, informiert. Es ist auch denkbar, dass die Erste-Hilfe-Station 2 das Vorliegen von Alarmereignissen eines Typs bestimmt, und das Rechensystem 4 das Vorliegen von Alarmereignissen eines anderen Typs. Diese Typen von Alarmereignissen können sich insbesondere hinsichtlich der Daten unterscheiden, die zur Bestimmung ihres Vorliegens herangezogen werden.

Ein Wiederbefüllen der Erste-Hilfe-Station 2 nach einer erfassten Entnahme eines Ersthilfsmittels sollte durch autorisiertes Personal erfolgen. Die Kommunikationseinrichtung 46 ist dazu ausgelegt, mittels Nahfeldkommunikation ein tragbares Identifikationsgerät 54, wie zum Beispiel einen Radiofunk-Transponder, zu erkennen. Nur wenn das Identifikationsgerät 54 erkannt wurde, wird eine Anzahl erfasster Entnahmen auf der Erste-Hilfe-Station 2 zurückgesetzt und ein eventuell vorhandenes Alarmereignis gelöscht. Das Zurücksetzen bzw. Löschen geschieht beispielsweise erst dann, wenn eine entsprechende Eingabe über die Benutzereingabeschnittstelle 42 empfangen wird und/oder wenn die Überwachungseinrichtung 16 ein Ersetzen eines entnommenen Ersthilfsmittels erfasst hat. Auch das Erkennen eines Identifikationsgeräts, das Zurücksetzen und/oder das Löschen eines Alarmereignisses kann von der Erste-Hilfe-Station 2 an das Rechensystem 4 gemeldet werden. So bleibt das Rechensystem 4 stets auf dem aktuellsten Stand über die aktuelle Einsatzbereitschaft der Erste-Hilfe-Station 2. Anstelle des Erkennens des Identifikationsgeräts 54 kann auch eine Autorisierung über die Benutzereingabeschnittstelle (z.B. durch Abfrage eines Passworts) vorgesehen sein.

Es versteht sich, dass die vorliegende Erfindung nicht auf das unter Bezugnahme auf Figur 1 beschriebene Ausführungsbeispiel beschränkt ist. So sind nicht alle zu diesem Ausführungsbeispiel beschriebenen Merkmale zwangsweise notwendig. Beispielsweise könnte die Erste-Hilfe-Station 2 anstelle mehrerer Ersthilfsmittel nur ein Ersthilfsmittel umfassen, z.B. den Verbandkasten 8 oder einen Defibrillator. Auch Abwandlungen des beschriebenen Ausführungsbeispiels sind möglich. So ist es beispielsweise möglich, anstelle zweier Abstandsmesseinrichtungen 30, 32 nur eine Abstandsmesseinrichtung oder einen Bewegungsmelder vorzusehen.

Anstelle oder zusätzlich zu dem unter Bezugnahme auf Fig. 1 beschriebenen Magnetfeldsensor (z.B. dem Sensor 20 oder/und dem Sensor 22) kann ein induktiver Sensor vorgesehen sein. In diesem Fall kann anstelle oder zusätzlich zu dem schon beschriebenen Magnet (z.B. dem Magnet 24 oder/und dem Magnet 26) ein leitfähiges oder/und ferromagnetisches Element vorgesehen sein. Dieses Element kann Teil des Ersthilfsmittels sein (z.B. ein Teil des Gehäuses des Verbandkastens 8 oder des Feuerlöschers 12). Auch der Einsatz von optischen Sensoren wie z.B. Kameras oder Lasersensoren zur Erfassung der Entnahme eines Ersthilfsmittels ist denkbar.

Alternativ oder zusätzlich zu den unter Bezugnahme auf Fig. 1 beschriebenen Sensoren können Funksensoren wie z.B. Radiofrequenzidentifikations- (RFID-) Sensoren, Nahfeldkommunikations- (NFC-) Sensoren, oder Bluetooth-Sensoren vorgesehen sein. Derartige Sensoren können als Funkempfänger bezeichnet werden. Hierbei kann ein von dem jeweiligen Sensor erfassbarer RFID-, NFC- bzw. Bluetooth-Sender an dem Ersthilfsmittel angeordnet sein. Ein Funkempfänger der Erste-Hilfe-Station kann dazu eingerichtet sein, von einem entsprechenden Funksender Informationen zu empfangen, sofern der Funksender von dem Funkempfänger weniger als einige Meter (z.B. < 5 Meter oder < 2 Meter) oder weniger als einige Zentimeter (z.B. < 20cm oder < 10cm) entfernt ist. Die von dem Funksender an den Funkempfänger übertragenen Informationen können auf der Erste-Hilfe-Station oder dem Rechensystem gespeichert werden. Die Informationen können ein Mindesthaltbarkeitsdatum eines Ersthilfsmittels umfassen. Beispielsweise ist ein RFID-Chip oder ein NFC-Tag an dem Ersthilfsmittel angeordnet und dient der Übermittlung des Mindesthaltbarkeitsdatums an den Funkempfänger. Zusätzlich oder alternativ können der Funkempfänger und der mit diesem kommunizierende Chip bzw. Tag zum Erfassen der Entnahme des Ersthilfsmittels eingesetzt werden, denn das von dem Funkempfänger empfangene Funksignal hängt von dessen Abstand zu dem das Funksignals aussendenden Funkempfänger ab. In diesem Fall haben der Funkempfänger und der entsprechende Funksender gewissermaßen eine Doppelfunktion.

Die vorliegend beschriebene Technik macht insbesondere zeit- und personalintensive Kontrollen von Erste-Hilfe-Stationen überflüssig. Auch ein zeitintensives Erstellen und Bereitstellen, sowie ein zeitintensives Kontrollieren von Betriebs- und Sicherheitsanweisungen kann mit der hier beschriebenen Technik entfallen. Ferner kann die hier beschriebene Technik ermöglichen, ein Einsammeln und eine Digitalisierung von Formularen eines papierbasierten Verbandbuchs zu vermeiden. Auch der Datenschutz kann durch die hier beschriebene Technik verbessert werden. Diese Technik ermöglicht es, einen Betreiber einer Erste-Hilfe-Station in Echtzeit über einen Status bezüglich der Ersthilfsmittel seiner Erste-Hilfe-Station zu informieren. Der Status kann z. B. über ein sogenanntes "Dashboard" eingesehen werden, das von einem Server oder in einer Cloud bereitgestellt wird. Auch kann die Technik einem Herausgeber ermöglichen, notwendige Betriebs- und Sicherheitsanweisungen aus der Ferne über einen Server bzw. eine Cloud bereitzustellen und zu aktualisieren. Die hier beschriebene Erste-Hilfe-Station kann als sogenannte "Plug-and-Play"-Lösung ausgestaltet sein, sodass sie auf einfache Weise an einem nahezu beliebigen Ort eingesetzt werden kann. Die hier beschriebene Technik kann es ermöglichen, bei Vorliegen eines Alarmereignisses relevante Personenkreise zu informieren, zum Beispiel mittels einer SMS- oder E-Mail-Nachricht. Die hier beschriebene Technik kann sicherstellen, dass nur autorisiertes Personal die Erste-Hilfe-Station mittels des Identifikationsgeräts zurücksetzen kann. Ein lokaler Alarm kann Mitarbeiter über eine Blockade der Erste-Hilfe-Station und/oder ein Vorliegen eines Alarmereignisses informieren. Es versteht sich, dass ein Fachmann der vorliegenden Offenbarung auch noch weitere Vorteile entnehmen kann.

## Patentansprüche

1. Erste-Hilfe-Station (2) zum Erfassen und Melden einer Entnahme zumindest eines Ersthilfsmittels (8; 10; 12), wobei die Erste-Hilfe-Station (2) aufweist:
- eine Halteeinrichtung (6) zum Halten zumindest eines Ersthilfsmittels (8; 10; 12) in der bzw. an der Erste-Hilfe-Station (2);
- eine Überwachungseinrichtung (16) zum Erfassen einer Entnahme des zumindest einen Ersthilfsmittels (8; 10; 12) aus der bzw. von der Erste-Hilfe-Station (2); und
- eine Kommunikationseinrichtung (46) zum Melden einer erfassten Entnahme über eine digitale Kommunikationsverbindung (48) an eine Überwachungsstelle.

2. Erste-Hilfe-Station (2) nach Anspruch 1, wobei die Überwachungseinrichtung (16) dazu eingerichtet ist, die Entnahme eines jeden Ersthilfsmittels (8; 10; 12) separat zu erfassen.

3. Erste-Hilfe-Station (2) nach Anspruch 1 oder 2, wobei das zumindest eine Ersthilfsmittel zumindest ein Medizinprodukt, insbesondere einen Verbandkasten (8), Verbandmaterial (10) und/oder einen Defibrillator umfasst.

4. Erste-Hilfe-Station (2) nach einem der Ansprüche 1 bis 3, ferner umfassend eine Benutzereingabeschnittstelle (42), die dazu eingerichtet ist, eine Benutzereingabe zu digitalisieren, wobei die Erste-Hilfe-Station (2) dazu eingerichtet ist, die digitalisierte Benutzereingabe an ein digitales Verbandbuch weiterzugeben.

5. Erste-Hilfe-Station (2) nach einem der Ansprüche 1 bis 4, wobei die Überwachungseinrichtung (16) einen Lichtsensor (18) umfasst und dazu eingerichtet ist, eine Entnahme zu erfassen, sobald von dem Lichtsensor detektiertes Licht eine vorbestimmte Eigenschaft aufweist.

6. Erste-Hilfe-Station (2) nach Anspruch 5, wobei der Lichtsensor (18) als Teil einer Lichtschranke der Überwachungseinrichtung (16) ausgebildet ist, wobei die Lichtschranke so angeordnet ist, dass sie bei einer Entnahme durchbrochen wird.

7. Erste-Hilfe-Station (2) nach einem der Ansprüche 1 bis 6, wobei die Überwachungseinrichtung (16) einen Magnetfeldsensor (20; 22) umfasst und dazu eingerichtet ist, eine Entnahme zu erfassen, sobald ein von dem Magnetfeldsensor (20; 22) detektiertes Magnetfeld eine vorbestimmte Eigenschaft aufweist.

8. Erste-Hilfe-Station (2) nach einem der Ansprüche 1 bis 7, wobei das zumindest eine Ersthilfsmittel zumindest eine Feuerlöschvorrichtung, insbesondere einen Feuerlöscher (12) und/oder eine Feuerlöschdecke umfasst.

9. Erste-Hilfe-Station (2) nach einem der Ansprüche 1 bis 8, wobei die Überwachungseinrichtung (16) einen Funksensor umfasst und dazu eingerichtet ist, eine Entnahme zu erfassen, sobald ein von dem Funksensor detektiertes Funksignal eine vorbestimmte Eigenschaft aufweist.

10. Erste-Hilfe-Station (2) nach einem der Ansprüche 1 bis 9, wobei die Überwachungseinrichtung (16) ferner dazu eingerichtet ist, eine Präsenz eines Objekts in einem dem zumindest einen Ersthilfsmittel (8; 10; 12) benachbarten Raumbereich zu detektieren.

11. Erste-Hilfe-Station (2) nach einem der Ansprüche 1 bis 10, welche dazu eingerichtet ist, bei Vorliegen eines eine benötigte Wartung der Erste-Hilfe-Station (2) indizierenden Alarmereignisses einen Alarm auszulösen oder auszugeben, wobei das Alarmereignis insbesondere dann vorliegt, wenn die Überwachungseinrichtung eine Entnahme erfasst hat, eine Anzahl von durch die Überwachungseinrichtung erfassten Entnahmen einen vorbestimmten Grenzwert überschreitet, ein Haltbarkeitsdatum eines Ersthilfsmittels (8; 10; 12) überschritten ist und/oder die Erste-Hilfe-Station (2) eine Meldung über ein Vorliegen des Alarmereignisses empfangen hat.

12. Erste-Hilfe-Station (2) nach einem der Ansprüche 1 bis 11, wobei die Kommunikationseinrichtung (46) ferner dazu ausgeführt ist, mittels Nahfeldkommunikation ein Identifikationsgerät (54) zu erkennen und basierend auf dem Erkennen eine Änderung einer Konfiguration der Erste-Hilfe-Station (2) zu bewirken.

13. Rechensystem (4) zum Verarbeiten von Meldungen der Erste-Hilfe-Station (2) gemäß einem der Ansprüche 1 bis 12, wobei das Rechensystem (4) eine Kommunikationsschnittstelle (50) für die digitale Kommunikationsverbindung (48) umfasst und dazu eingerichtet ist, von der Erste-Hilfe-Station (2) über die Kommunikationsschnittelle (50) eine Meldung einer erfassten Entnahme des zumindest einen Ersthilfsmittels (8; 10; 12) zu empfangen und basierend auf der Meldung der erfassten Entnahme eine vorbestimmte Aktion durchzuführen.

14. Rechensystem (4) nach Anspruch 13, wobei die vorbestimmte Aktion ein Bestimmen umfasst, ob ein Alarmereignis vorliegt, welches eine benötigte Wartung der Erste-Hilfe-Station (2) indiziert.

15. Gesamtsystem (100), umfassend:
- eine Erste-Hilfe-Station (2) nach einem der Ansprüche 1 bis 12; und
- zumindest ein Ersthilfsmittel (8; 10; 12) und/oder ein Rechensystem (4) nach Anspruch 13 oder 14.

## Geänderte Patentansprüche

### Geänderte Patentansprüche gemäss Regel 137(2) EPÜ.

1. Erste-Hilfe-Station (2) zum Erfassen und Melden einer Entnahme zumindest eines Ersthilfsmittels (8; 10; 12), wobei die Erste-Hilfe-Station (2) aufweist:
- eine Halteeinrichtung (6) zum Halten zumindest eines Ersthilfsmittels (8; 10; 12) in der bzw. an der Erste-Hilfe-Station (2);
- eine Überwachungseinrichtung (16) zum Erfassen einer Entnahme des zumindest einen Ersthilfsmittels (8; 10; 12) aus der bzw. von der Erste-Hilfe-Station (2); und
- eine Kommunikationseinrichtung (46) zum Melden einer erfassten Entnahme über eine digitale Kommunikationsverbindung (48) an eine Überwachungsstelle,
wobei die Überwachungseinrichtung (16) ferner dazu eingerichtet ist, ein Eintreten oder Hineingreifen einer Person in einen dem zumindest einen Ersthilfsmittel (8; 10; 12) benachbarten Raumbereich und/oder eine Präsenz eines Objekts in einem dem zumindest einen Ersthilfsmittel (8; 10; 12) benachbarten Raumbereich zu detektieren.

2. Erste-Hilfe-Station (2) nach Anspruch 1, wobei die Überwachungseinrichtung (16) dazu eingerichtet ist, die Entnahme eines jeden Ersthilfsmittels (8; 10; 12) separat zu erfassen.

3. Erste-Hilfe-Station (2) nach Anspruch 1 oder 2, wobei das zumindest eine Ersthilfsmittel zumindest ein Medizinprodukt, insbesondere einen Verbandkasten (8), Verbandmaterial (10) und/oder einen Defibrillator umfasst.

4. Erste-Hilfe-Station (2) nach einem der Ansprüche 1 bis 3, ferner umfassend eine Benutzereingabeschnittstelle (42), die dazu eingerichtet ist, eine Benutzereingabe zu digitalisieren, wobei die Erste-Hilfe-Station (2) dazu eingerichtet ist, die digitalisierte Benutzereingabe an ein digitales Verbandbuch weiterzugeben.

5. Erste-Hilfe-Station (2) nach einem der Ansprüche 1 bis 4, wobei die Überwachungseinrichtung (16) einen Lichtsensor (18) umfasst und dazu eingerichtet ist, eine Entnahme zu erfassen, sobald von dem Lichtsensor detektiertes Licht eine vorbestimmte Eigenschaft aufweist.

6. Erste-Hilfe-Station (2) nach Anspruch 5, wobei der Lichtsensor (18) als Teil einer Lichtschranke der Überwachungseinrichtung (16) ausgebildet ist, wobei die Lichtschranke so angeordnet ist, dass sie bei einer Entnahme durchbrochen wird.

7. Erste-Hilfe-Station (2) nach einem der Ansprüche 1 bis 6, wobei die Überwachungseinrichtung (16) einen Magnetfeldsensor (20; 22) umfasst und dazu eingerichtet ist, eine Entnahme zu erfassen, sobald ein von dem Magnetfeldsensor (20; 22) detektiertes Magnetfeld eine vorbestimmte Eigenschaft aufweist.

8. Erste-Hilfe-Station (2) nach einem der Ansprüche 1 bis 7, wobei das zumindest eine Ersthilfsmittel zumindest eine Feuerlöschvorrichtung, insbesondere einen Feuerlöscher (12) und/oder eine Feuerlöschdecke umfasst.

9. Erste-Hilfe-Station (2) nach einem der Ansprüche 1 bis 8, wobei die Überwachungseinrichtung (16) einen Funksensor umfasst und dazu eingerichtet ist, eine Entnahme zu erfassen, sobald ein von dem Funksensor detektiertes Funksignal eine vorbestimmte Eigenschaft aufweist.

10. Erste-Hilfe-Station (2) nach einem der Ansprüche 1 bis 9, welche dazu eingerichtet ist, bei Vorliegen eines eine benötigte Wartung der Erste-Hilfe-Station (2) indizierenden Alarmereignisses einen Alarm auszulösen oder auszugeben, wobei das Alarmereignis insbesondere dann vorliegt, wenn die Überwachungseinrichtung eine Entnahme erfasst hat, eine Anzahl von durch die Überwachungseinrichtung erfassten Entnahmen einen vorbestimmten Grenzwert überschreitet, ein Haltbarkeitsdatum eines Ersthilfsmittels (8; 10; 12) überschritten ist und/oder die Erste-Hilfe-Station (2) eine Meldung über ein Vorliegen des Alarmereignisses empfangen hat.

11. Erste-Hilfe-Station (2) nach einem der Ansprüche 1 bis 10, wobei die Kommunikationseinrichtung (46) ferner dazu ausgeführt ist, mittels Nahfeldkommunikation ein Identifikationsgerät (54) zu erkennen und basierend auf dem Erkennen eine Änderung einer Konfiguration der Erste-Hilfe-Station (2) zu bewirken.

12. Rechensystem (4) zum Verarbeiten von Meldungen der Erste-Hilfe-Station (2) gemäß einem der Ansprüche 1 bis 11, wobei das Rechensystem (4) eine Kommunikationsschnittstelle (50) für die digitale Kommunikationsverbindung (48) umfasst und dazu eingerichtet ist, von der Erste-Hilfe-Station (2) über die Kommunikationsschnittelle (50) eine Meldung einer erfassten Entnahme des zumindest einen Ersthilfsmittels (8; 10; 12) und eine Meldung (i) eines Eintretens oder Hineingreifens einer Person in einen dem zumindest einen Ersthilfsmittel (8; 10; 12) benachbarten Raumbereich oder (ii) einer Präsenz eines Objekts in einem dem zumindest einen Ersthilfsmittel (8; 10; 12) benachbarten Raumbereich zu empfangen und basierend auf den Meldungen eine vorbestimmte Aktion durchzuführen.

13. Rechensystem (4) nach Anspruch 12, wobei die vorbestimmte Aktion ein Bestimmen umfasst, ob ein Alarmereignis vorliegt, welches eine benötigte Wartung der Erste-Hilfe-Station (2) indiziert.

14. Gesamtsystem (100), umfassend:
- eine Erste-Hilfe-Station (2) nach einem der Ansprüche 1 bis 11; und
- zumindest ein Ersthilfsmittel (8; 10; 12) und/oder ein Rechensystem (4) nach Anspruch 12 oder 13.
